Europäisches Patentamt

(19) European Patent Office

Office européen des brevets

(11) Numéro de publication: **0 084 748**
**B1**

(12) **FASCICULE DE BREVET EUROPEEN**

(45) Date de publication du fascicule du brevet:
08.05.85

(51) Int. Cl.⁴: **C 07 C 1/20**, B 01 J 29/18,
B 01 J 29/38, C 01 B 33/28

(21) Numéro de dépôt: **82402251.1**

(22) Date de dépôt: **09.12.82**

(54) Production d'hydrocarbures à partir de méthanol en présence de catalyseurs du type zéolithe.

(30) Priorité: **04.01.82 FR 8200011**

(43) Date de publication de la demande:
**03.08.83 Bulletin 83/31**

(45) Mention de la délivrance du brevet:
**08.05.85 Bulletin 85/19**

(84) Etats contractants désignés:
**AT BE CH DE FR GB IT LI LU NL SE**

(56) Documents cités:
**DE - A - 2 935 863**
**FR - A - 2 019 913**
**GB - A - 1 379 257**
**US - A - 4 052 472**

**Le dossier contient des informations techniques présentées postérieurement au dépôt de la demande et ne figurant pas dans le présent fascicule.**

(73) Titulaire: **SOCIETE CHIMIQUE DE LA GRANDE PAROISSE, AZOTE ET PRODUITS CHIMIQUES, 8, rue Cognacq-Jay, F-75007 Paris (FR)**

(72) Inventeur: **Hamon, Christian, 47, Chemin des Dames, F-44600 Saint-Nazaire (FR)**
Inventeur: **Bandiera, Jean, 345, Balmont Est La Duchère, F-69009 Lyon (FR)**
Inventeur: **Senes, Michel, La Clotière 11, avenue des Flandres, F-44500 La Baule (FR)**

(74) Mandataire: **Bouton Neuvy, Liliane et al, L'Air liquide, Société Anonyme pour L'Etude et L'Exploitation des Procédés Georges Claude 75, Quai d'Orsay, F-75321 Paris Cedex 07 (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention réalisée avec la coopération de l'Institut de Recherches sur la Catalyse, concerne la production d'hydrocarbures à partir de méthanol en présence de catalyseurs zéolithiques du type mordénite, et tout particulièrement l'obtention d'hydrocarbures insaturés ayant de 2 à 5 atomes de carbone, dits hydrocarbures oléfiniques légers.

Le méthanol est accessible à partir de sources carbonées d'origine non pétrolière, telles le charbon pour la voie gaz de synthèse et les végétaux par tansformation de cellulose. Ainsi, cet alcool est appelé à devenir une matière de base essentielle de la chimie en vue de la fabrication des grands intermédiaires de synthèse. L'obtention d'hydrocarbures oléfiniques légers à partir de méthanol est donc une direction de recherche intéressante.

La réaction de conversion du méthanol en hydrocarbures a fait l'objet de nombreux travaux. Les caalyseurs mis en oeuvre appartiennent le plus souvent à la classe des zéolithes. Ces silicoaluminates cristallins conviennent particulièrement à ce type de réaction en raison de leur caractère acide et de leur structure parfaitement déterminée dont les diamètres des canaux intercristallins sont du même ordre de grandeur que ceux de la plupart des molécules organiques.

Cependant, un des inconvénients essentiels deces procédés d traitement catalytique du méthanol sur zéolithes cristallines est constitué par la désactivation rapide du catalyseur ar des dépôts d'origine carbonée, ce qui empêche tout développement industriel dans des conditions économiques normales.

On an recherché un catalyseur zéolithique modifié permettant de produire essentiellement des hydrocarbures insaturés ayant pour l'essentiel de 2 à 5 atomes de carbone, et dont la durée de vie catalytique est notablement augmentée.

On sait que la mordénite est l'un des silicates zéolithiques les plus riches en silice. Le procédé décrit dans le brevet français 1 411 753 permet de syntétiser une mordénite sodique à petits pores de 4 à 5Å, dont la formule du motif élémentaire est $Na_1Al_7 Si_{40} O_{94}$, 24 $H_2O$. Le rapport Si/Al (atomique) est voisin de 6 sur la forme sodique.

Antérieurement, notamment selon l'enseignement du brevet français 2 019 913, on sait que des mordénites soumises à des cycles multiples alternés débutant par un traitement par la vapeur d'eau puis de reflux par un acide, ont été proposées comme compositions catalytiques pour les conversions des hydrocarbures. Par contact catalytique avec des mordénites ayant un rapport silice/alumine supérieur à 15, désaluminées par soumission à des cycles multiples alternés débutant par un traitement par la vapeur d'eau à la pression atmosphérique puis par un traitement à l'acide chlorhydrique, selon le brevet US 4 052 472, on convertit du méthanol et/ou des diméthyléther en un mélange d'hydrocarbures contenant plus de 50% d'hydrocarbures en $C_9$ et $C_{10}$ et en aromatiques lourds.

La mordénite acide, obtenue par traitement thermique, sous air à 650°C, de la forme ammonium est très active dans la transformation du méthanol en hydrocarbures mais sa durée de vie est très courte. Après moins de 30 minutes de réaction, il a été constaté que le taux de conversion du méthanol initialement de 100 devient inférieur à 1%.

Or, il a été observé que la durée de vie de la mordénite engagée dans une réaction catalytique de traitement du méthanol est très substantiellement accrue lorsque le rapport Si/Al augmente. Et il a été découvert que l'utilisation d'une zéolithe du type mordénite synthétique dont le rapport Si/Al (atomique) est supérieur à 80 est déterminante dans l'obtention d'hydrocarbures à partir de méthanol, quant à la répartition des produits obtenus et surtout quant à la stabilité du catalyseur dans le temps. Les catalyseurs du type mordénite désaluminée dans lesquels le rapport Si/Al (atomique) est compris entre 100 et 150 se sont révélés particulièrement avantageux dans la transformation du méthanol en hydrocarbures insaturés.

Le catalyseur selon l'invention est avantageusement applicable à un procédé catalytique, en phase hétérogène, de transformation du méthanol en hydrocarbures éthyléniques, ayant pour l'essentiel de 2 à 5 atomes de carbone.

Selon ce procédé, le méthanol est introduit dans un gaz porteur, pouvant être indifféremment l'azote, l'hydrogène, le monoxyde de carbone ou le dioxyde de carbone seuls ou en mélange. Le procédé est mis en oeuvre en exploitation continue dans un réacteur avec circulation du mélange gazeux : méthanol-gaz diluant sur le catalyseur.

La réaction de transformation du méthanol est conduite à une pression comprise entre la pression atmosphérique et 20 bars, à une température comprise enre 350 et 550°C.

La concentration en méthanol dans le mélange gazeux entrant est avantageusement comprise enre 5 et 60% en volume, de préférence 30 à 50%; le débit volumétrique horaire (LSVH) est compris entre 1 et 5 litres de méthanol liquide par litre de catalyseur et par heure.

Après séparation des hydrocarbures formés, le gaz porteur est rcyclé; le méthanol non transformé et le diméthyléther sont également recyclables.

Les catalyseurs désaluminés utilisés dans la transformation du méthanol sont obtenus par un traitement désaluminant.

La désalumination de la mordénite peut être effectuée selon deux méthodes distinctes qui conduisent à l'obtention de catalyseurs désaluminés (type I) et doublement désaluminés (type II et III).

Dans la description on a adopté la notation suivante : NaZ désignant la forme sodique de la mordénite, NH₄Z la forme ammonium, HZ la forme acide obtenue par décomposition de la forme ammonium. On peut effectuer par exemple un échange préalable des cations Na⁺ par des ions NH₄⁺ en traitant la mordénite sodique par une solution aqueuse d'un sel d'ammonium; la mordénite acide non désaluminée étant obtenue par traitement thermique de la forme ammonium.

La double désalumination de la mordénite, du type désalumination hydrothermique, qui consiste en une série de traitements acides et hydrothermiques alternés conduit à partir de la mordénite forme sodique aux catalyseurs de type II, et à partir de la mordénite forme ammonium aux catalyseurs de type III; la série de traitements commençant toujours par un traitement acide et se terminant par un traitement acide.

Le traitement acide dans l'un ou l'autre cas a les caractéristiques suivantes. Les acides minéraux forts conviennent pour ce type d traitement, tels les acides sulfurique e chlorhydrique, de préférence l'acide chlorhydrique avantageusement à concentration comprise entre 2N et 9N. La température de traitement est comprise entre 60 et 90°C, l'opération étant effectuée sous agitation et à reflux, la teneur en mordénite étant voisine de 400 grammes par litre de milieu acide. Le traitement est poursuivi pendant une durée de quelques heures.

Le traitement hydrothermique consiste à porter la mordénite à une température comprise entre 550 et 680°C, de préférence entre 600 et 650°C, et à l'y maintenir pendant plusieurs heures, sous mélanges air-vapeur d'eau, à pression atmosphérique. La concentration en vapeur d'eau (volume) est comprise entre 2,5 et 60%, et de préférence enre 5 et 35%.

Les catalyseurs de type II et III ont une teneur en sodium résiduel inférieur à 0,1% en poids.

Il a été constaté que l'extraction d'aluminium de la mordénite est largement facilitée par le traitement hydrothermique. L'évolution du rapport Si/Al selon les deux voies de désalumination a été étudiée en fonction du nombre de cycles. Par cycle, on désigne un traitement acide suivi d'un traitement hydrothermique pour les catalyseurs de type II ou III; de plus, il a été constaté qu'un traitement final acide est avantageux.

Le traitement acide correspond à titre d'exemple à un traitement par HCl 6N à 80°C pendant trois heures, et le traitement hydrothermique est conduit à 650°C pendant cinq heures. Les essais ont été effectués sur 200 g de mordénite pour les catalyseurs de type II ou III, le rapport Si/Al dans les mordénites NaZ et NH₄Z de départ était de 5,7. On a obtenu les résultats suivants:

| nombre de cycle | Si/Al (type II ou III) |
|---|---|
| 2 | 46 |
| 3 | 82 |

Il a été observe que l'augmentation de la température du traitement hydrothermique au-dessus de 700°C a un effet néfaste sur la stabilité du catalyseur.

Par durée de vie des catalyseurs, on entend la durée pendant laquelle l'activité est sensiblement constante, dans le type de convesion du métanol en hydrocarbures, cette conversion étant totale, ce temps est celui au bout duquel commence l'apparition du diméthyléther et simultanément du méthanol non transformé dans le mélange réactionnel.

Les performances des catalyseurs doublement désaluminés (type II et III) sont supérieures à celles de catalyseurs désaluminés par voie chimique acide selon la technique connue (type I). Le rapport Si/Al optimal est compris entre 100 et 150. On n'observe pas dans cet intervalle de modifications significatives dans l'activité et la distribution des produits obtenus. Contrairement aux catalyseurs décrits précédemment, la durée de vie dans cet intervalle est peu sensible au rapport Si/Al. D'autre part, les catalyseurs obtenus selon cette voie à partir de NH₄Z ou HZ (type III) présentent une activité et une durée de vie supérieures à celles des catalyseurs obtenus à partir de NaZ (type II), les distributions de produits sont peu différentes. Dans les conditions de fonctionnement en réacteur pilote, leur durée de vie est respectivement supérieure à 15h (II) et à 30h (III). Ils sont très sélectifs en éthyléniques C₂—C₅, qui représentent plus de 75% du méthanol transformé. Le produit majoritaire est le propène. La distribution des produits est fonction pour un lit catalytique donné du temps de travail du catalyseur et des facteurs cinétiques de la réaction (temps de contact pression de méthanol, température).

Il a été constaté que l'augmentation de la pression partielle de méthanol accroit la proportion d'aromatiques méthylés; principalement l'hexaméthyl benzène au détriment des benzène, toluène et xylènes.

Une élèvation de température en augmentant le taux de conversion a pour conséquence, d'une part une diminution des aromatiques méthylés, d'autre part une augmentation des hydrocarbures légers (éthylène-propène) au détriment des hydrocarbures plus lourds (C₅ · C₆). L'augmentation de la pression totale de 1 à 20 bars absolus accroit la proportion d'hydrocarbures insaturés dans la série aliphatique.

Les caractéristiques des différents types de catalyseurs utilisables dans l'invention figurent dans le tableau

Caracteristiques des differents types de mordenite

| Type | composition % Poids | | | $\Delta P_{700°C}$ | Si/Al (atome) | Paramètres (Å) | | |
|------|------|------|------|------|------|------|------|------|
| | $SiO_2$ | $Al_2O_3$ | $Na_2O$ | | | a | b | c |
| NaZ | 70,5 | 10,4 | 6,3 | 12,8 | 5,7 | 18,137 | 20,423 | 7,505 |
| NH₄Z | 74,9 | 10,7 | <0,1 | 14,3 | 5,9 | 18,152 | 20,292 | 7,49 |
| II | 97,1 | 1 | <0,1 | 1,9 | 82,4 | 18,082 | 20,242 | 7,445 |
| III | 94,7 | 0,9 | <0,1 | 4,4 | 89,3 | 18,085 | 20,197 | 7,445 |

— La mordénite cristallise dans le système orthorhombique (Groupe Cmcm), $\Delta P$ comprend $H_2O + NH_3$.

En raison de la désactivation des catalyseurs dans un temps limité, on peut mettre en oeuvre le procédé de l'invention avec régénération périodique du catalyseur, par combustion à l'oxygène du carbone déposé. On effectue cette opération par de l'air dilué avec un gaz inerte, tel l'azote à une température comprise entre 450 et 600°C. La vitesse volumétrique horaire dans les conditions normales de température et de pression (VVH) est comprise entre 5000 et 15 000 h⁻¹. La teneur en $O_2$, voisine de 2% (Volume) au début de la régénération est ensuite augmentée progressivement en prenant soin de ne pas dépasser 600°C. La durée de cette opération est de quelques heures, environ 3 heures. Le catalyseur ainsi régénéré retrouve son activité et sa durée de vie initiale. Les catalyseurs ont été testés à la fois en micro réacteur et réacteur pilote.

En micro réacteur, le catalyseur est sous forme de poudre, la masse utilisée pouvant varier de 100 à 400 mg. La réaction est effectuée à pression atmosphérique. Le gaz porteur est l'hydrogène; la pression de méthanol, ajustée par un système de saturateurs-condenseurs, est limitée à environ 136 millibars. En réacteur pilote, le catalyseur est sous forme de pastilles; la mise en forme étant obtenue par incorporation d'un liant argileux à raison de 10% en poids. Le volume utilisé varie de 20 à 80 cm3.

Il est donné ci-après quelques exemples non limitatifs de préparation de catalyseurs et de leur application dans le procédé de l'invention.

Dans ces exemples les pourcentages relatifs aux hydrocarbures formés sont exprimés par rapport au méthanol transformé.

$C_n^+$ désigne la somme des hydrocarbures (aromatiques exceptés) à n atomes de carbone et plus;
$\sum C_4H_8$ désigne la somme des butènes;
$(i+1) C_4H_8$ désigne l'isobutène plus le butène-1;
$(t+c) C_4H_8$ désigne le transbutène plus le cis butène-2;
$C_1 - C_4$ désigne la somme des hydrocarbures ayant de 1 à 4 atomes de carbone;
$S = 1-4$ représente le pourcentage d'oléfines dans la série $C_1 - C_4$;
$A_r$ désigne les Aromatiques et DME le diméthyléther.

## Exemple 1

### (Comparatif) Mordénite acide HZ

400 g de mordénite sodique à petits pores en poudre, de formule $Na_7Al_7$, $Si_{40}O_{94}$, 24 $H_2O$ sont ajoutés dans 1 litre d'une solution aqueuse contenant 100 g de nitrate d'ammonium. Le mélange est agité à une température de 60°C pendant 4 heures. La mordénite est ensuite récupérée par filtration puis lavée à l'eau déminéralisée jusqu'à pH7. La zéolithe est alors soumise à un traitement identique sans séchage préalable. Cette opération est renouvelée une 3éme fois. Le solide est alors séché à 100°C puis calciné sous air à 650°C pendant 3h. On obtient ainsi la forme acide dénommée HZ. Les cations Na⁺ sont progressivement échangés au cours de ces échanges par des ions NH₄⁺. La teneur en sodium résiduel, initialement de 4,6% est après ces traitements successifs respectivement de 1,1 — 0,21 et inférieure à 0,1% en poids du produit sec. Le rapport Si/Al (atomique) est peu différent de celui de la mordénite de départ soit 5,7.

## Application

100 mg de mordénite acide HZ sont placés dans un micro-réacteur en verre situé dans un four. Le catalyseur est prétraité in situ à 500°C sous air à un débit de 2Nl/h pendant 2h. Le lit catalytique est ensuite traversé par un mélange gazeux de méthanol et d'hydrogène. Les conditions de cet essai et la répartition des produits obtenus figurent ci-après. La durée de vie de ce catalyseur est très courte. Après moins de 30 minutes de réaction le taux de transformation du méthanol en hydrocarbures est inférieur à 1%, pour les conditions de fonctionnement suivantes: à pression atmosphérique, température de 450°C, débit de méthanol de 100 mg/h (soit une pression de 37,8 millibars de méthanol), le débit d'hydrogène plus de méthanol gaz étant de 2Nl/h.

— Répartition des produits fabriqués après 2 minutes de réaction en% de méthanol transformé:

Tableau 1-1

| Méthanol non transformé | 0 |
|---|---|
| Diméthyléther | 0 |
| $C_1 - C_4$ | 75 |
| Aromatiques légers $< C_9$ | 5 |
| $C_5^+$ | $< 1$ |
| $\Delta$ | $\sim 20$ |

— Répartition $C_1 - C_4$

| $CH_4$ | 1,6 |
|---|---|
| $C_2H_4$ | 35 |
| $C_2H_6$ | 8,9 |
| $C_3H_6$ | 11,1 |
| $C_3H_8$ | 36,5 |
| iso $C_4H_{10}$ | 3 |
| n $C_4H_{10}$ | 2,8 |
| $\sum C_4H_8$ | 1,1 |
| | 100,0 |

## Exemple 2

### Mordénite doublement désaluminée

#### 2.a. à partir de mordénite NaZ

Dans un ballon de 2,5 l on introduit successivement 400 g de mordénite sodique en poudre et 1 l d'une solution aqueuse d'HCl de normalité 6N; la suspension est agitée à reflux pendant 5 h à une température de 90°C. La mordénite est séparée par filtration puis lavée avec 10 litres d'eau déminéralisée. Le pH est alors voisin de 5. On soumet ensuite ce catalyseur à un traitement hydrothermique, à pression atmosphérique, dans un four horizontal balayé par un courant d'air à raison de 250 Nl/h. La température est montée progressivement à 650°C à raison de 150°C/h. La vapeur est introduite dans l'air à partir de 300°C. Sa teneur est ajustée au moyen d'un saturateur. Le débit d'eau est dans nos conditions voisin de 80 g/h soit une teneur en volume dans le mélange air-vapeur d'eau d'environ 28%. Après 5 h de palier à 650°C dans ces conditions, l'alimentation du four est coupée. Le refroidissement s'effectue à la vitesse du four sous balayage air-vapeur d'eau; à partir de 300°C, la vapeur d'eau est supprimée. La mordénite est ensuite reprise par HCl dans les mêmes conditions puis soumise à un traitement hydrothermique identique. L'opération (traitement acide et hydrothermique) est renouvelée une 3ème fois. La teneur en $Al_2O_3$ du produit est de 1% et le rapport Si/Al (atomique) de 82,4. Les caractéristiques de cette mordénite désaluminée de type II, composition % en poids, et paramètres de maille en Å sont indiquées ci-après:

| Type | Composition % poids | | | $\Delta P_{700°C}$ | Si/Al (atome) | paramètres (Å) | | |
|---|---|---|---|---|---|---|---|---|
| | $SiO_2$ | $Al_2O_3$ | $Na_2O$ | | | a | b | c |
| II | 97,1 | 1 | $< 0,1$ | 1,9 | 82,4 | 18,082 | 20,242 | 7,445 |

## Application

### — A — Mise en oeuvre en microréacteur

On soumet 100 g de ce ctalyseur sous forme de poudre à des essais catalytiques pour la conversion du méthanol en micro-réacteur à pression atmosphérique, la température de réaction est de 450°C, le gaz porteur est l'hydrogène à un débit de 2Nl/h, celui du méthanol étant d 100 mg/h soit une pression de $CH_3OH$ de 37,8 millibars. On a procédé à l'étude de l'évolution de l'activité du catalyseur dans le temps.

Tableau 2-1

| Durée de fonctionnement | $CH_3OH$ non transformé | $C_1-C_4$ | Ar | $C_5^+$ | DME | $S_1^{=4}$ |
|---|---|---|---|---|---|---|
| 2' | 2 | 69 | 9 | 20 | < 1 | 78 |
| 2h | 3 | 68 | 8 | 22 | 1 | 86 |
| 5h | 4 | 67 | 7 | 20 | 2 | 89 |
| 7h | 6 | 65 | 7 | 19 | 3 | 90 |
| 22h | 9 | 62 | 6 | 19 | 4 | 95 |

Tableau 2-2

Répartition $C_1-C_4$. Chiffres exprimés en $CH_3OH$ transformé en %.

| Durée de fonctionnement | $CH_4$ | $C_2H_4$ | $C_2H_6$ | $C_3H_6$ | $C_3H_8$ | $iC_4H_{10}$ | $(i+1)$ $C_4H_8$ | $nC_4H_{10}$ | $t+c$ $C_4H_8$ |
|---|---|---|---|---|---|---|---|---|---|
| 2' | 1 | 13 | <0,1 | 51 | 11 | 9 | 8 | 0,8 | 6,2 |
| 5h | 1 | 3,5 | <0,1 | 64 | 3,5 | 5,5 | 13,1 | 0,3 | 8,5 |
| 22h | 1 | 2 | ~0,1 | 63 | 1 | 2,9 | 16,5 | 0,2 | 13,4 |

Parmi les aromatiques, le produit principal est l'hexaméthyl-benzène, représentant plus de 50% des aromatiques formés. Il se forme essentiellement des dérivés méthylés dont la teneur augmente avec le degré de substitution.

On a étudié l'influence de la température de transformation du méthanol, en présence du catalyseur doublement désaluminé à partir de NaZ, après 26 heures de marche à 450°C, dans les conditions de marmarche en microréacteur.

Tableau 2-3

Taux de transformation du méthanol %

| t°C | $CH_3OH$ non transformé | $C_1-C_4$ | $S_1^{=4}$ | Ar | DME | $C_5^+$ |
|---|---|---|---|---|---|---|
| 400 | 23 | 41 | 96 | 3 | 18 | 15 |
| 450 | 15 | 55 | 95 | 3 | 9 | 18 |
| 500 | 9 | 63 | 93 | 4 | 5 | 19 |

# 0 084 748

B — Mise en oeuvre en réacteur pilote

Dans un réacteur droit vertical, on charge 40 cm3 du catalyseur pastillé (3 × 2 mm) contenant 10% en poids d'un liant argileux. La masse du catalyseur est de 27 g soit 24,3 g de mordénite. Il est prétraité in situ, sous un débit d'air de 250 Nl/h à 500°C pendant 2 heures. Après cette période d'activation, le réacteur est alimenté en continu de haut en bas avec un mélange méthanol-azote préchauffé à 400°C. Le méthanol injecté par une pompe est, avant mélange avec le vecteur $N_2$, vaporisé dans un préchauffeur.

On a fait varier les paramètres cinétiques dans la réaction: température, débit, pression partielle de méthanol ainsi que la pression totale de la pression atmosphérique à 15 bars absolus.

L'ensemble des résulats est consigné dans les tableaux ci-après.

Tableau 2-4

Influence du débit total ($N_2 + CH_3OH$) à pression méthanol constante. Température moyenne 475°C, pression du méthanol 109,3 millibars. Evolution du méthanol non transformé en hydrocarbures.

| Débit $N_2 + CH_3OH$ Nl/h | durée fonctionnement | $CH_3OH$ non transformé + diméthyléther | LSVH $h^{-1}$ | temps contact réel | VVH gaz $h^{-1}$ |
|---|---|---|---|---|---|
| 280 | 1 h | 7 | 1,25 | 0,19 s | 7 000 |
| | 3 h | 9 | | | |
| | 6 h | 15 | | | |
| 840 | 1 h | 28 | 3,75 | 0,06 s | 21 000 |
| | 3 h | 29 | | | |
| | 6 h | 38 | | | |

LSVH $h^{-1}$: débit de méthanol liquide (1) litre catalyseur/heure.

Tableau 2-5

Influence de la pression partielle de méthanol. Evolution du méthanol transformé en hydrocarbures en fonction du temps. Débit $CH_3OH$ constant 40 g/h, tc = 450°C (moyenne), vecteur $N_2$.

| Débit $N_2$Nl/h | p. $CH_3OH$ mb | VVh gaz $h^{-1}$ | temps contact réel | t fonction | $CH_3OH$ %*) transformé |
|---|---|---|---|---|---|
| 500 | 57,3 | 13 250 | 0,11 | 1 h | 72 |
| | | | | 6 h | 66 |
| | | | | 24 h | 58 |
| 250 | 108 | 7 000 | 0,22 | 2 h | 90 |
| | | | | 5 h | 83 |
| | | | | 13 h | 67 |
| 150 | 169 | 4 500 | 0,36 | 1 h | 98 |
| | | | | 5 h | 68 |
| | | | | 10 h | 40 |

*) ce chiffre correspond au taux de transformation du $CH_3OH$ en hydrocarbures, il ne comprend pas le diméthyléther.

— 2-b A partir de mordénite $NH_4Z$

200 g de mordénite sodique à petits pores sont soumis à un traitement identique à celui décrit dans l'exemple 1; on obtient par cette méthode la forme ammonium $NH_4Z$. Le produit est séché à l'étuve à 100°C puis désaluminé selon la procédure décrite dans l'exemple précédent (3 cycles successifs de traitements acides et hydrothermiques alternés).

7

**0 084 748**

Le produit obtenu a une teneur en $Al_2O_3$ de 0,9% et un rapport Si/Al d'environ 90. Sa composition et les paramètres de maille sont les suivants:

| Type | Composition % Poids | | | $\Delta P_{700°C}$ | Si/Al (atome) | Paramètres (Å) | | |
|------|------|------|------|------|------|------|------|------|
| | $SiO_2$ | $Al_2O_3$ | $Na_2O$ | | | a | b | c |
| III | 94,7 | 0,9 | <0,1 | 4,4 | 89,3 | 18,085 | 20,197 | 7,445 |

Ce catalyseur a été soumis à des tests en micro réacteur et réacteur pilote dans les conditions décrites précédemment. L'ensemble des résultats indiqués ci-dessous concerne le réacteur pilote.

Ce catalyseur présente par rapport à celui désaluminé à partir de NaZ des performances supérieures quant à l'activité et la durée de vie.

On a également fait varier les paramètres cinétiques de la réaction: température, pression partielle de méthanol débit. Les résultats sont dans l'ensemble concordants avec ceux obtenus avec le catalyseur précédent. La répatition des produits est voisine; par contre la vitesse de désactivation est beaucoup moins sensible à l'augmentation de la pression partielle de méthanol avec ce catalyseur qu'avec le précédent.Ainsi, avec un débit de méthanol de 40 g/h, la durée de vie du catalyseur (conversion $CH_3OH$ en hydrocarbures >90%) est supérieure à 40 heures avec un débit $N_2$ de 250 Nl/h soit une pression de méthanol de 108 millibars: elle est encore de 35 heures lorsque le débit d'azote est abaissé à 50 Nl/h soit une pression de méthanol de 380 millibars.

La sélectivité évolue en fonction du temps. En particulier, la production d'éthylène et d'isobutane diminue au profit du propène et de l'isobutène. La coupe $C_1—C_4$ représente plus de 70% du méthanol transformé; la sélectivité en insaturés étant supérieure à 90%. Le produit majoritaire est le propène. Dans la série des aromatiques on a essentiellement des dérivés méthylés dont la teneur augmente avec le degré de substitution; le produit majoritaire est l'hexaméthylbenzène.

On an également fait varier la pression totale de la pression atmosphérique à 20 bars absolus. La durée de vie du catalyseur n'est pas améliorée. Par contre l'on modifie sensiblement la répartition des produits fabriqués au profit des saturés.

Comparaison des mordénites »doublement désaluminées« à partir de NaZ (type II) et $NH_4Z$ (type III).

Conditions de fonctionnement: Réacteur pilote volume 40 cm3, catalyseur en pastilles $3\times3$ mm, température 450°C, pression atmosphérique, débit gaz porteur azote $N_2$250Nl/h, débit $CH_3OH$ 40 g/h.

Evolution du taux de transformation du méthanol en hydrocarbures, le diméthyléther n'étant pas comptabilisé dans les chiffres indiqués.

Tableau 2-6

| durée de fonctionnement catalyseur type III | | durée de fonctionnement catalyseur type II | |
|------|------|------|------|
| 2 h | 98,3 | 1 h | 93 |
| 18 h | 97,5 | 3 h | 91 |
| 27 h | 96,2 | 6 h | 90 |
| 34 h | 93,5 | 12 h | 86 |
| 42 h | 91,2 | 20 h | 77 |

Dans les mêmes conditions de fonctionnement mises en oeuvre avec le catalyseur doublement désaluminé du type III, on a étudié la répartition moyenne des produits fabriqués sur 35 heures de réaction.

8

Tableau 2-7

|  | Taux de transformation de méthanol % |
|---|---|
| $CH_3OH$ non transformé | 2,3 |
| Diméthyléther | 1,2 |
| $C_1$ | 1,3 |
| $C_2H_4$ | 5,9 |
| $C_2H_6$ | <0,1 |
| $C_3H_6$ | 49,5 |
| $C_3H_8$ | 0,7 |
| $C_4 S$ | 2,9 |
| $C_4 S^=$ | 14,5 |
| $C_5 S$ | 1 |
| $C_5 S^=$ | 9 |
| $C_6{}^+$ | 6 |
| Aromatiques | 5,2 |
| CoKe + CO + $CO_2$ | $\sim$0,5 |
|  | 100,0 |

Tableau 2-8

| $C_4$ | Répartition % |
|---|---|
| Isobutane | 12,1 |
| n-butane | 1 |
| butène-1 | 7,1 |
| Isobutène | 39 |
| Trans 2-butène | 23,3 |
| cis 2-butène | 17,5 |
|  | 100,00 |

Tableau 2-9

| $C_5$ | Répartition % |
|---|---|
| Méthyl-3 butène-1 | 3,4 |
| Isopentane | 7,5 |
| pentène-1 | 5,4 |
| méthyl-2 butène-1 | 18,5 |
| n-pentane | 4 |
| trans-2 pentène | 16,2 |
| cis -2 pentène | 8,4 |
| méthyl-2 butène-2 | 35,6 |
| cyclopentane + cyclopentène | 1 |
|  | 100,00 |

De plus, on a étudié l'influence de la pression totale sur la distribution des produits $C_1 - C_5$ (% $CH_3OH$ transformé) après 2 heures de réaction conduite en réacteur pilote avec un volume de catalyseur de 40 cm3 en pastilles 3 × 3 mm, les débits de $N_2$ étant de 750 Nl/h et de $CH_3OH$ 120 g/h.

Tableau 2-10

| Pression | $CH_4$ | $C_2H_4$ | $C_2H_6$ | $C_3H_6$ | $C_3H_8$ | $iC_4H_{10}$ | $(i+1)$ $C_4H_8$ | $nC_4H_{10}$ | butè-ne-2 | $C_5S$ | $C_5S^=$ |
|---|---|---|---|---|---|---|---|---|---|---|---|
| Atm. | 0,6 | 3,8 | 0,1 | 31 | 0,8 | 1,1 | 7,1 | 0,1 | 3,5 | 1 | 7,2 |
| 10 bars | 1,3 | 3,9 | 0,1 | 25 | 2,2 | 5,2 | 5,5 | 0,8 | 3,2 | 2,8 | 4,4 |

La production d'hydrocarbures par kilogramme de catalyseur en 35 heures de fonctionnement, 57,6 kg de méthanol ayant traversé le catalyseur serait la suivante:

|  | Kg | en $> CH_2$ |
|---|---|---|
| Méthanol non converti | 1,33 | 0,52 |
| diméthyl éther | 0,69 | 0,30 |
| $CH_4$ | 0,75 | 0,33 |
| $C_2H_4$ | 3,4 | 1,48 |
| $C_3H_6$ | 28,51 | 12,46 |
| $C_3H_8$ | 0,40 | 0,18 |
| $C_4S$ | 1,67 | 0,73 |
| $C_4S^=$ | 8,35 | 3,65 |
| $C_5S$ | 0,58 | 0,25 |
| $C_5S^=$ | 5,18 | 2,27 |
| $C_6^+$ | 3,46 | 1,51 |
| Aromatiques | 3 | 1,31 |
| $Coke + CO + CO_2$ | 0,12 | 0,12 |
|  | 57,6 | 27,17 kg hydrocarbures |

## Exemple 3

600 g de mordénite sodique sont transformés en forme ammonium selon la procédure décrite dans l'exemple 1. La température de chaque traitement est de 80°C. La teneur en Na est inférieure à 0,05% en poids. Ce produit est soumis à une double désalumination, telle que décrite dans l'exemple 2a, mettant en oeuvre successivement des traitements chlorhydriques et hydrothermiques. La température du traitement acide est de 90°C et celle du traitement hydrothermique de 650°C; la teneur en vapeur d'eau (%Vol) du mélange air-vapeur d'eau est de 15%. La teneur en $Al_2O_3$ du produit sec est après 2 cycles de 0,9%. On soumet cette mordénite désaluminée à un traitement chlorhydrique complémentaire dans des conditions identiques au précédent (HCL 6N-90°C). La teneur en $Al_2O_3$ est alors de 0,7% (Si/Al > 100) = 120. Ce catalyseur a été soumis à des tests en réacteur pilote selon la mise en oeuvre décrite dans les exemples 2a et b.

Les conditions de fonctionnement sont les suivantes:

| $CH_3OH$: | 40 g/h |
|---|---|
| Porteur $N_2$: | 50 Nl/h |
| Pression atmosphérique | |
| Température moyenne: | 470°C. |

La transformation du méthanol en hydrocarbures est totale. La répartition des produits obtenus est voisine de celle consignée dans le tableau 2-7 avec toutefois une diminution légère de la teneur en éthylène. La durée de vie du catalyseur est augmentée par rapport au catalyseur de l'exemple 2b où la désalumination se termine par un traitement hydrothermique. Après 50 h de fonctionnement le taux de transformation du méthanol en hydrocarbures est supérieur à 90%. Taux de transformation du méthanol:

| $CH_4$: | 1,2 |
|---|---|
| $C_2H_4$: | 3,2 |
| $C_2H_6$: | 0,1 |
| $C_3H_6$: | 53 |

0 084 748

C_3H_8:                        1,1
C_4H_8:                        16,8
C_4H_{10}:                     4,3
C_5H_{10}:                     7,8
C_5H_{12}:                     1,2
C_6^+:                         6
Aromatiques:                   5
»coke« + CO + CO_2:            0,3

## Exemple 4

200 g de mordénite désaluminée sont préparés selon la procédure décrite dans l'exemple 2b:

Préparation de $NH_4Z$ à partir de $NaZ$ suivi d'une désalumination par des traitements HCl et hydrothermiques alternés. Les conditions du traitement acide sont identiques; par contre, la teneur en vapeur d'eau dans le mélange air-vapeur d'eau est abaissée à 2,5% (Vol), les autres paramètres (température, temps, débit) étant inchangés. Le produit obtenu après 3 cycles a une teneur en $Al_2O_3$ de 1,2%. Ce catalyseur a été testé en réacteur pilote dans des conditions identiques après mise en forme (pastilles de diamètre 3 mm, 10% liant). Les conditions de fonctionnement sont les suivantes:

$CH_3OH$:                      40 g/h;
$N_2$:                         50 Nl/h;
Pression atmosphérique;
température moyenne:           470° C.

La durée de vie de ce catalyseur est comprise entre 10 et 15 h. Après 20 h de réaction, le taux de transformation du méthanol en hydrocarbures est inférieur à 50%. (Le complément à 100 étant du diméthyléther et du méthanol non transformé.)

La répartition des produits est sensiblement différente de l'exemple précédent (2b), particulièrement au niveau de la coupe $C_4$ et $C_5$ où l'on note une augmentation des saturés. Le produit majoritaire de la coupe $C_4$ est l'isobutane. Il décroit au cours du temps au profit de l'isobutène mais beaucoup moins que dans les autres cas. Il est indiqué ci-dessous, l'évolution comparative du rapport iso $C_4H_{10}$/iso $C_4H_8$

| Temps de réaction | Exemple | Exemple 2b |
|---|---|---|
| 1 h | 3,8 | 0,9 |
| 4 h | 2,2 | 0,45 |
| 10 h | 1,1 | 0,2 |

La répartition des produits obtenus (par atomes de carbone) est par contre très peu modifiée. Le propène reste le produit majoritaire (>50%/méthanol transformé).

## Revendications

1. Procédé de production d'hydrocarbures insaturés ayant de 2 à 5 atomes de carbone dits hydrocarbures oléfiniques légers, à partir de méthanol en présence de catalyseur zéolithiques du type mordénite désaluminée, caractérisé en ce qu'on fait passer le méthanol gazéifié introduit dans un gaz porteur, la concentration du méthanol dans le mélange entrant étant comprise entre 5 et 60% en volume, à travers un lit catalytique à base de mordénite synthétique du type dit désaluminée dont le rapport Si/Al (atomique) est supérieur à 80, de préférence compris entre 100 et 150; ladite mordénite désaluminée étant obtenue à partir de mordénite synthétique soumise à une série de traitements acides et hydrothermiques alternés; dans la phase traitement acide, la mordénite est soumise à un traitement en milieu acide concentré, de préférence de normalité entre 2 et 9N, à température comprise entre 60 et 90° C, sous agitation pendant quelques heures; dans la phase traitement hydrothermique, la mordénite est portée à une température comprise entre 550 et 680° C, de préférence 600 à 650° C, pendant plusieurs heures, à pression atmosphérique, sous un mélange air-vapeur d'eau dont le débit air-vapeur d'eau dans les conditions normales de température et de pression est compris entre 500 et 1250 l/h pour une masse de catalyseur de 1 kg; la concentration de vapeur d'eau en volume étant

11

comprise entre 2,5 et 60%, de préférence 5 et 35%; la série de traitements commençant par un traitement acide et se terminant par un traitement acide; le débit de méthanol étant compris entre 1 et 5 litres de méthanol liquide par litre de catalyseur et par heure, la réaction étant effectuée à température comprise entre 350 et 550° C, sous pression pouvant atteindre 20 bars.

2. Procédé de production d'hydrocarbures à partir de méthanol, selon la revendication 1, caractérisé en ce que la concentration du méthanol entrant est comprise entre 30 et 50% en volume.

3. Procédé de production d'hydrocarbures à partir de méthanol, selon une quelconque des revendications 1 à 2, caractérisé en ce que le méthanol non transformé est recyclé, éventuellement conjointement avec le diméthyléther formé, également recyclé.

4. Procédé de production d'hydrocarbures à partir de méthanol selon une quelconque des revendications 1 à 3, caractérisé en ce que le procédé est mis en oeuvre avec régénération périodique du catalyseur par combustion à l'oxygène dilué avec un gaz inerte, à température comprise entre 450 et 600° C pendant quelques heures, la vitesse volumétrique horaire dans les conditions normales de température et de pression étant comprise entre 5000 et 15 000 h$^{-1}$. La teneur en oxygène étant de l'ordre de 2% en volume au début de la régénération, avec augmentation progressive sans dépasser la température de 600° C.

5. Procédé de désalumination hydrothermique de mordénite soumise à une série de traitements acides et hydrothemique alternés, caractérisé en ce que la série de traitement commence par un traitement acide et se termine par un traitement acide; dans la phase traitement acide, la mordénite est soumise à un traitement en milieu acide concentré, de préférence de normalité entre 2 et 9N, à température comprise entre 60 et 90° C, sous agitation pendant quelques heures; dans la phase traitement hydrothermique, la mordénite est portée à une température comprise entre 550 et 680° C, de préférence 600 à 650° C, pendant plusieurs heures, à pression atmosphérique, sous un mélane air-vapeur d'eau dont le débit air-vapeur d'eau dans les conditions normales de température et de pression est compris entre 500 et 1250 l/h pour une masse de catalyseur de 1 kg; la concentration de vapeur d'eau en volume étant comprise entre 2,5 et 60%, de préférence 5 et 35%.

6. Mordénite doublement désaluminée obtenue par le procédé selon la revendication 5 à partir de mordénite sodique.

7. Mordénite doublement désaluminée obtenue par le procédé selon la revendication 5 à partir de mordénite forme ammonium.

## Patentansprüche

1. Verfahren zur Herstellung von ungesättigten Kohlenwasserstoffen mit 2 bis 5 Kohlenstoffatomen, sogenannter leichter Olefinkohlenwasserstoffe, mit Hilfe von Methanol in Gegenwart eines zeolithischen Katalysators vom entaluminierten Mordenittyp, dadurch gekennzeichnet, daß man das verdampfte, in ein Trägergas eingeführte Methanol, wobei die Methanolkonzentration in dem Gemisch zwischen 5 und 60 Volumen-% beträgt, durch eine katalytische Schicht auf der Grundlage von synthetischem Mordenit des besagten entaluminierten Typs, dessen Si/Al-Verhältnis (Atomverhältnis) über 80, vorzugsweise zwischen 100 und 150 ist, gehen läßt, wobei der entaluminierte Mordenit aus synthetischem Mordenit erhalten wurde, der einer Reihe von alternierenden sauren und hydrothermalen Behandlungen unterzogen wurde, wobei in der sauren Behandlungsphase der Mordenit einer Behandlung in konzentriertem saurem Milieu, vorzugsweise mit einer Normalität zwischen 2 und 9 N, einer Temperatur zwischen 60 und 90° C unter Rühren während mehrerer Stunden ausgesetzt wurde und der Mordenit in der hydrothermalen Behandlungsphase auf eine Temperatur zwischen 550 und 680° C, vorzugsweise zwischen 600 und 650° C, während mehrerer Stunden bei Atmosphärendruck unter einem Luft-Wasserdampfgemisch, dessen Durchsatz unter Normalbedingungen der Temperatur und des Druckes zwischen 500 und 1250 l/h pro Kilogramm Katalysator lag gebracht wurde, wobei die Wasserdampfkonzentration zwischen 2,5 und 60 Volumen-%, vorzugsweise zwischen 5 und 35 Volumen-% lag und die Reihe von Behandlungen mit einer sauren Behandlung begann und mit einer sauren Behandlung endete, der Methanoldurchsatz zwischen 1 und 5 l flüssigen Methanols je Liter Katalysator und je Stunde liegt und die Umsetzung bei einer Temperatur zwischen 350 und 550° C bei einem Druck, der 20 bar erreichen kann, durchgeführt wird.

2. Verfahren zur Herstellung von Kohlenwaserstoffen mit Hilfe von eintretendem Methanol nach Anspruch 1, dadurch gekennzeichnet, daß die Methanolkonzentration zwischen 30 und 50 Volumen-% liegt.

3. Verfahren zur Herstellung von Kohlenwasserstoffen mit Hilfe von Methanol nach einem der Ansprüche 1 und 2, dadurch gekennzeichnet, daß das nichtumgewandelte Methanol zurückgeführt wird, gegebenenfalls gemeinsam mit dem gebildeten, gleichfalls zurückgeführten Dimethyläther.

4. Verfahren zur Herstellung von Kohlenwasserstoffen mit Hilfe von Methanol nach einem der Ansprüche 1 bis 3, dadurch gekennzeichnet, daß das Verfahren unter periodischer Regenerierung des Katalysators durch Verbrennung mit Sauerstoff, der mit einem Inertgas verdünnt ist, bei einer Temperatur zwischen 450 und 600° C während mehrerer Stunden unter allmählicher Steigerung, ohne die Temperatur von 600°C zu überschreiten, durchgeführt wird, wobei die stündliche volumetrische Geschwindigkeit unter Normalbedingungen der Temperatur und des Druckes zwischen 5000 und 15 000 h$^{-1}$ liegt und der Sauerstoffgehalt bei Beginn der Regenerierung in der Größenordnung von 2 Volumen-% liegt.

5. Verfahren zur hydrothermalen Entaluminierung von Mordenit, der einer Reihe von alternierenden sauren und hydrothermalen Behandlungen unterzogen wird, dadurch gekennzeichnet, daß die Behandlungsreihe mit einer sauren Behandlung beginnt und mit eine sauren Behandlung endet, in der sauren Behandlungsphase der Mordenit einer Behandlung in konzentriertem saurem Milieu, vorzugsweise einer Normalität zwischen 2 und 9 N, bei einer Temperatur zwischen 60 und 90°C unter Rühren während mehrerer Stunden unterzogen wird, in der hydrothermalen Behandlungsphase der Mordenit auf eine Temperatur zwischen 550 und 680°C, vorzugsweise zwischen 600 und 650°C, während mehrerer Stunden bei Atmosphärendruck unter einem Luft-Wasserdampfgemisch gebracht wird, wobei der Luft-Wasserdampf-Durchsatz unter den Normalbedingungen der Temperatur und des Druckes zwischen 500 und 1250 l/h für eine Katalysatormasse von 1 kg liegt und die Wasserdampfkonzentration zwischen 2,5 und 60, vorzugsweise zwischen 5 und 35 Volumen-% ist.

6. Doppelt entaluminierter Mordenit, erhalten durch das Verfahren nach Anspruch 5 aus Natriummordenit.

7. Doppelt entaluminierter Mordenit, erhalten durch das Verfahren nach Anspruch 5 aus Mordenit in der Ammoniumform.

**Claims**

1. Process for the production of unsaturated hydrocarbons having 2 to 5 carbon atoms, called light olefinic hydrocarbons, by means of methanol in presence of a zeolitic catalyst of the type of dealuminified mordenite, characterized in that the vaporized methanol introduced into a carrier gas, the concentration of methanol in the mixture being between 5 and 60 percent by volume, is passed through a catalytic bed ased on synthetic mordenite of the said dealuminified type the Si/Al-ratio (atomic) of which is above 80, preferably between 100 and 150, the said dealuminified mordenite being obtained by means of synthetic mordenite subjected to a series of alternating acidic and hydrothermal treatments, in the acidic treatment phase the mordenite is subjected to a treatment in the concentrated acidic medium, preferably of a normality between 2 and 9 N, at a temperature between 60 and 90°C with agitation during several hours, in the hydrothermal treatment phase the mordenite is brought to a temperature between 550 and 680°C, preferably between 600 and 650°C, during several hours at atmospheric pressure under an air-steam mixture the throughput of which air-steam under the normal conditions of temperature and pressure is between 500 and 1250 l/h for a catalyst mass of 1 kg, the concentration by volume of steam being between 2,5 and 60 percent, preferably between 5 and 35 percent, the series of treatments beginning by an acidic treatment and terminating by an acidic treatment, the throughput of methanol being between 1 and 5 l of liquid methanol per liter of catalyst and per hour, the reaction being carried out at a temperature between 350 and 550°C, whereby the pressure can reach 20 bars.

2. Process for the production of hydrocarbons by means of entering methanol according to claim 1, chararacterized in that the concentration of the entering methanol is between 30 and 50 percent by volume.

3. Process for the production of hydrocarbons by means of methanol according to one of the claims 1 to 2, characterized in that the non-transformed methanol is recycled, optionally together with the formed dimethyl ether also recycled.

4. Process for the production of hydrocarbons by means of methanol according to one of the claims 1 to 3, characterized in that the process is carried out with periodic regeneration of the catalyst by combustion with oxygen diluted with an inert gas at a temperature between 450 and 600°C during several hours with progressive augmentation without exceeding the temperature of 600°C, the volumetric hourly rate at the normal conditions of temperature and pressure being between 5000 and 15 000 h$^{-1}$ and te content of oxygen being in the order of 2 percent by volume at the beginning of the regeneration.

5. Process of hydrothermal dealuminification of mordenite subjected to a series of alternating acidic and hydrothermal treatments, chararacterized in that the series of treatments begins with an acidic treatment and terminates with an acidic treatment, in the acidic treatment phase the mordenite is subjected to a treatment in a concentrated acidic medium, preferably of a normality between 2 and 9 N, at a temperature between 60 and 90°C, with agitation during several hours, in the hydrothermal treatment phase the mordenite is brought to a temperature between 550 and 680°C, preferably between 600 and 650°C during several hours at atmospheric pressure under an air-steam mixture, the throughput of the air-steam under the normal conditions of temperature and pressure being between 500 and 1250 l/h for a catalyst mass of 1 kg, the steam concentration by volume being between 2,5 and 60 percent, preferably between 5 and 35 percent.

6. Double-dealuminified mordenite obtained by the process according to claim 5 by means of sodium mordenite.

7. Double-dealuminified mordenite obtained by the process according to claim 5 by means of mordenite in the ammonium form.